# EUROPÄISCHE PATENTSCHRIFT

(11) **EP 4 507 781 B1**
(45) Veröffentlichungstag und Bekanntmachung des Hinweises auf die Patenterteilung: **11.06.2025**
(21) Anmeldenummer: 23748006.6
(22) Anmeldetag: 17.07.2023
(51) Int. Cl.: A61N 5/06, A61M 35/00

(54) **GERÄT ZUR BEHANDLUNG EINES GEWEBEABSCHNITTS EINES MENSCHEN ODER EINES TIERES**
DEVICE FOR TREATING A TISSUE PORTION OF A HUMAN OR ANIMAL
DISPOSITIF DE TRAITEMENT D'UN MORCEAU DE TISSU D'UN ÊTRE HUMAIN OU D'UN ANIMAL

(30) Priorität: 27.07.2022 DE 102022118867
(43) Veröffentlichungstag der Anmeldung: 19.02.2025
(73) Patentinhaber: Klose, Thomas, 56068 Koblenz (DE); Steier, Liviu, Needham, Massachusetts 02492 (US)
(72) Erfinder: Klose, Thomas, 56068 Koblenz (DE); Steier, Liviu, Needham, Massachusetts 02492 (US)
(74) Vertreter: Bernsmann, Falk
(86) Internationale Anmeldenummer: PCT/EP2023/069832
(87) Internationale Veröffentlichungsnummer: WO 2024/022876

(56) Entgegenhaltungen:
- WO-A1-2016/010765
- US-A1- 2011 040 235
- US-A1- 2011 123 958
- US-A1- 2011 144 410
- US-A1- 2016 051 834
- US-A1- 2017 036 002
- US-A1- 2017 209 484
- US-A1- 2018 193 622
- US-A1- 2019 133 908

## Beschreibung

### Technisches Gebiet

Die Erfindung betrifft ein Gerät zur Behandlung eines Gewebeabschnitts (W), insbesondere einer Wunde, eines Menschen oder eines Tieres, das Gerät umfassend eine Applikationsvorrichtung zur Applikation einer ersten Zusammensetzung, einer zweiten Zusammensetzung und einer dritten Zusammensetzung zur Behandlung des Gewebeabschnitts auf den Gewebeabschnitt und eine Belichtungsvorrichtung zur Belichtung der auf den Gewebeabschnitt applizierten Zusammensetzungen mit Licht und eine Tragvorrichtung, wobei die Tragvorrichtung die mindestens eine Applikationsöffnung und die Austrittsfläche trägt.

### Stand der Technik

Unter der photodynamischen Therapie (PDT) versteht man ein Verfahren zur Behandlung unter anderem von Tumoren und anderen Gewebeveränderungen wie beispielsweise Gefäßneubildungen mit Licht in Kombination mit einer lichtaktivierbaren Substanz, einem so genannten Photosensibilisator, und im Gewebe vorhandenem Sauerstoff. Dazu wird dem Patienten ein solcher, primär nicht toxischer Sensibilisator oder einer seiner Stoffwechselvorläufer entweder systemisch oder lokal verabreicht, der sich aufgrund bestimmter Eigenschaften des Tumors oder der Gewebeveränderung selektiv im Tumor oder der Gewebeveränderung anreichert. Nach einer gewissen Wartezeit wird der Tumor oder die Gewebeveränderung mit Licht geeigneter Wellenlänge bestrahlt. Dabei werden durch photophysikalische Prozesse toxische Substanzen, vor allem reaktive Sauerstoffspezies, erzeugt, die den Tumor oder die Gewebeveränderung schädigen.

Bei der photodynamischen Desinfektion, auch antibakterielle photodynamische Therapie (aPDT) oder photoaktivierte Desinfektion genannt, handelt es sich um ein unter anderem in der Zahnmedizin zur unterstützenden Behandlung von bakteriellen Infektionen bei Parodontitis verwendetes Verfahren (Clinical Oral Investigations (2022) 26:3005-3010 https://doi.org/10.1007/s00784-021-04282-z; Der Hautarzt (2005) 11:1048-1055 https://doi.org/10.1007/s00105-005-0977-7; Der Hautarzt (2021) 1:27-33 https://doi.org/10.1007/s00105-020-04737-6). Wie bei der photodynamischen Therapie kommt dabei Licht in Kombination mit einer lichtaktivierbaren Substanz, einem so genannten Photosensibilisator und vorhandenem Luftsauerstoff zum Einsatz.

Üblicherweise umfasst die photodynamische Desinfektion folgende Schritte:
a. Anfärben der Mikroorganismen mit einem Photosensibilisator,
b. Einwirken lassen des Photosensibilisators während einer von Indikation und Schweregrad der Erkrankung abhängigen Einwirkzeit,
c. Spülen und Entfernen des überschüssigen Photosensibilisators,
d. Belichten des Photosensibilisators, mit einer von Indikation und Schweregrad der Erkrankung abhängigen Wellenlänge, Intensität und Belichtungsdauer.

Die einzelnen Schritte werden dabei mit unterschiedlichen Geräten durchgeführt, und gegebenenfalls müssen die Schritte auch je nach Indikation mit unterschiedlichen Geräten durchgeführt werden, beispielsweise um ein Belichten mit unterschiedlichen Wellenlängen zu ermöglichen. Ferner sind in der Regel zusätzliche Geräte für weitere Schritte der Wundbehandlung, beispielsweise zur Anregung der Wundheilung, notwendig. Durch die Vielzahl verwendeter Geräte ist die Behandlung aufwändig und fehleranfällig, insbesondere ist es schwierig, bei einer lokal begrenzten Wunde alle Behandlungsschritte exakt am Ort der Wunde durchzuführen.

Außerdem führen bekannte Methoden der photodynamischen Desinfektion nicht immer zu einer vollständigen Desinfektion, sodass eine zusätzliche Behandlung mit anderen Desinfektionsmethoden notwendig ist, was den Behandlungsaufwand weiter steigert.

### Technische Aufgabe

Die Aufgabe der Erfindung ist es, vielseitige Vorrichtungen zur einfachen und wirksamen Behandlung eines Gewebeabschnitts, insbesondere einer Wunde, eines Menschen oder eines Tieres zu schaffen.

### Technische Lösung

Die vorliegende Erfindung stellt ein Gerät gemäß Anspruch 1 bereit, das die technische Aufgabe löst. Vorteilhafte Ausgestaltungen sind Gegenstand der abhängigen Ansprüche.

Das Gerät ist zur Behandlung eines Gewebeabschnitts, insbesondere einer Wunde, eines Menschen oder eines Tieres ausgelegt. Der Gewebeabschnitt kann beispielsweise ein Hautabschnitt oder ein Schleimhautabschnitt, insbesondere ein Abschnitt einer Mundschleimhaut sein. Bei der Wunde kann es sich um eine äußere oder innere Gewebeverletzung, beispielsweise um eine Gewebeverletzung in einer Mundhöhle, eines Menschen oder eines Tiers handeln. Die Gewebeverletzung kann beispielsweise durch einen chirurgischen Eingriff verursacht sein.

Das Gerät umfasst eine Applikationsvorrichtung zur Applikation einer ersten Zusammensetzung, einer zweiten Zusammensetzung und einer dritten Zusammensetzung zur Behandlung des Gewebeabschnitts auf den Gewebeabschnitt, wobei die Applikationsvorrichtung mindestens eine Applikationsöffnung zur Abgabe der Zusammensetzungen aus der Applikationsvorrichtung an den Gewebeabschnitt umfasst.

Das Gerät umfasst eine Belichtungsvorrichtung zur Belichtung der auf den Gewebeabschnitt applizierten Zusammensetzungen mit Licht, wobei die Belichtungsvorrichtung eine Austrittsfläche zum Austritt des Lichts aus der Belichtungsvorrichtung umfasst.

Das Gerät umfasst eine Tragvorrichtung, wobei die Tragvorrichtung die mindestens eine Applikationsöffnung und die Austrittsfläche trägt. Weitere Teile der Applikationsvorrichtung und/oder der Belichtungsvorrichtung können separat von der Tragvorrichtung angeordnet sein, sodass die Tragvorrichtung möglichst klein und leicht sein kann. Die außerhalb der Tragvorrichtung angeordneten Teile können durch eine Anzahl von Leitungen, beispielsweise zur Leitung der Zusammensetzungen und/oder des Lichts, mit der Tragvorrichtung verbunden sein. Die Leitungen sind vorzugsweise biegsam, sodass sie eine den Gewebeabschnitt behandelnde Behandlungsperson und/oder die Erreichbarkeit des Gewebeabschnitts nicht behindern.

### Vorteilhafte Wirkungen

Dadurch, dass die mindestens eine Applikationsöffnung und die Austrittsfläche von der Tragvorrichtung getragen sind, können die Applikation und die Belichtung mit der gleichen Tragvorrichtung durchgeführt werden. Folglich ist die Behandlung des Gewebeabschnitts besonders einfach und örtlich besonders exakt durchführbar.

Insbesondere wird durch die Tragvorrichtung vermieden, dass die Zusammensetzungen außerhalb des Behandlungsbereichs appliziert werden. Dadurch wird eine besonders kleine Menge der Zusammensetzungen benötigt, und mögliche Nebenwirkungen werden vermieden. Ein Kontakt einer, insbesondere erfindungsgemäßen oder hypochlorige Säure enthaltenden, Zusammensetzung mit Schleimhäuten oder offenen Wunden kann beispielsweise zu Irritationen führen.

Außerdem wird dadurch, dass die mindestens eine Applikationsöffnung und die Austrittsfläche von der gleichen Tragvorrichtung getragen sind, eine definierte Positionierung und Ausrichtung der Austrittsfläche relativ zu der mindestens einen Applikationsöffnung festgelegt. Dadurch kann sichergestellt werden, dass die Belichtung genau dort erfolgt, wo die Zusammensetzungen appliziert wurden. Dadurch ist eine besonders effiziente Behandlung möglich, und Nebenwirkungen durch eine Belichtung von Gewebe außerhalb eines Applikationsbereichs der Zusammensetzungen werden vermieden.

Dadurch, dass die Applikationsvorrichtung zur Applikation einer ersten Zusammensetzung, einer zweiten Zusammensetzung und einer dritten Zusammensetzung zur Behandlung des Gewebeabschnitts auf den Gewebeabschnitt ausgelegt ist, können mit dem gleichen Gerät, insbesondere gleichzeitig, bis zu drei voneinander unterschiedliche Zusammensetzungen verwendet werden. Folglich werden die Wirksamkeit der Behandlung und die Vielseitigkeit des Gerätes gesteigert, ohne den Aufwand für die Behandlung zu erhöhen.

### Beschreibung der Ausführungsarten

Das Gerät umfasst vorzugsweise eine Spülvorrichtung zur Spülung des Gewebeabschnitts mit einem Spülfluid, wobei die Spülvorrichtung mindestens eine von der Tragvorrichtung getragene Spülöffnung zur Abgabe des Spülfluids aus der Spülvorrichtung an den Gewebeabschnitt umfasst. Die mindestens eine Applikationsöffnung kann mit der mindestens einen Spülöffnung identisch sein.

Das Gerät umfasst vorzugsweise eine Absaugvorrichtung zur Absaugung des Spülfluids von dem Gewebeabschnitt, wobei die Absaugvorrichtung mindestens eine von der Tragvorrichtung getragene Absaugöffnung zur Aufnahme des Spülfluids von dem Gewebeabschnitt in die Absaugvorrichtung umfasst.

Weitere Teile der Spülvorrichtung und/oder der Absaugvorrichtung außer der mindestens einen Spülöffnung und/oder der mindestens einen Absaugöffnung können separat von der Tragvorrichtung angeordnet sein, sodass die Tragvorrichtung möglichst klein und leicht sein kann. Die außerhalb der Tragvorrichtung angeordneten Teile können durch eine Anzahl von Leitungen, beispielsweise zur Leitung des Spülfluids, mit der Tragvorrichtung verbunden sein. Die Leitungen sind vorzugsweise biegsam, sodass sie eine den Gewebeabschnitt behandelnde Behandlungsperson nicht behindern.

Dadurch, dass auch die mindestens eine Spülöffnung und/oder die mindestens eine Absaugöffnung von der Tragvorrichtung getragen ist, können auch die Spülung und/oder die Absaugung mit der Tragvorrichtung durchgeführt werden, sodass die Behandlung besonders einfach und das Gerät besonders vielseitig einsetzbar ist. Außerdem definiert die Tragvorrichtung die Position und Ausrichtung der Spülöffnung und/oder der Absaugöffnung relativ zu der Applikationsöffnung, sodass die Spülung und/oder die Absaugung exakt am Ort der Applikation der Zusammensetzungen durchgeführt werden können, sodass die Behandlung besonders zielgenau und effizient erfolgt.

Ein Abstand der Spülöffnung von der Absaugöffnung beträgt vorzugsweise von 1 mm bis 10 mm, bevorzugt von 1 mm bis 8 mm, besonders bevorzugt von 1 mm bis 5 mm. Die genannten Abstände erlauben eine besonders effiziente Spülung des Gewebeabschnitts.

Die Applikationsvorrichtung umfasst vorzugsweise eine Fluss-Steuereinheit zur, insbesondere voneinander unabhängigen, Steuerung jeweils eines Flusses der drei Zusammensetzungen auf den Gewebeabschnitt. Mit Hilfe der Fluss-Steuereinheit kann auf einfache und zuverlässige Weise jeweils eine zur Behandlung optimale Menge der Zusammensetzungen auf den Gewebeabschnitt appliziert werden.

Die Fluss-Steuereinheit umfasst bevorzugt ein Ventil, besonders bevorzugt ein Rückschlagventil, eine Pumpe und/oder einen Druckerzeuger, besonders bevorzugt mit einer Druckgaspatrone. Das Rückschlagventil kann vorteilhafterweise verhindern, dass Fremdstoffe von dem Gewebeabschnitt in die Applikationsvorrichtung eindringen und die Zusammensetzungen kontaminieren. Die Pumpe und der Druckerzeuger können einen Transport der Zusammensetzungen auf den Gewebeabschnitt unterstützen und somit deren Applizierung vereinfachen.

Die Applikationsvorrichtung umfasst vorzugsweise eine Zerstäubungseinheit zur Zerstäubung der ersten Zusammensetzung, der zweiten Zusammensetzung und/oder der dritten Zusammensetzung bei der Applikation auf den Gewebeabschnitt. Durch die Zerstäubung kann die Wirksamkeit der zerstäubten Zusammensetzung durch eine vergrößerte Oberfläche der Zusammensetzung erhöht werden. So wurde beispielsweise für eine wässrige Lösung von hypochloriger Säure eine erhöhte Desinfektionswirkung festgestellt (BOECKER, D., et al. Safe, Effective, and cost-efficient air cleaning for populated rooms and entire buildings based on the disinfecting power of vaporized hypochlorous acid. International Journal of Health and Medical Engineering, 2022, year 16, no. 12, p. 209-213). Die Zerstäubungseinheit kann beispielsweise so gestaltet sein wie die in US 9 573 148 B2 beschriebene Zerstäubungseinheit.

Die Applikationsvorrichtung ist vorzugsweise zur Aufnahme einer austauschbaren Kartusche enthaltend die erste Zusammensetzung, die zweite Zusammensetzung und/oder die dritte Zusammensetzung ausgelegt. Mit Hilfe der Kartusche können die Zusammensetzungen besonders einfach in die Applikationsvorrichtung eingefüllt, nachgefüllt und gegen andere Zusammensetzungen ausgetauscht werden. Dadurch ist das Gerät besonders einfach und vielseitig verwendbar.

Die Applikationsvorrichtung umfasst bevorzugt mindesten eine Kanüle, besonders bevorzugt jeweils eine Kanüle, zur Entnahme der Zusammensetzungen aus der Kartusche. Mit der Kanüle kann die Kartusche automatisch angestochen werden, sobald die Kartusche in die Applikationsvorrichtung eingesetzt ist. Somit kann die Kartusche besonders einfach und ohne Gefahr, ihren Inhalt zu verunreinigen, geöffnet werden. Wenn für jede Zusammensetzung jeweils eine Kanüle vorgesehen ist, wird dadurch eine Vermischung der Zusammensetzungen, die deren Wirksamkeit beeinträchtigen könnte, verhindert.

Die Applikationsvorrichtung ist vorzugsweise zur Aufnahme mehrerer Kartuschen ausgelegt, wobei die Applikationsvorrichtung vorzugsweise ein Auswahlelement, bevorzugt ein Karussell, zur Auswahl einer der Kartuschen zur Applikation der in der ausgewählten Kartusche enthaltenen Zusammensetzungen umfasst. Dadurch können für unterschiedliche Indikationen unterschiedliche Zusammensetzungen in der Applikationsvorrichtung bereitgehalten und auf einfache Weise zur Verwendung bei der Desinfektion ausgewählt werden.

Das Gerät kann eine Herstellungsvorrichtung zur Herstellung zumindest einer der Zusammensetzungen umfassen. Die Herstellungsvorrichtung kann beispielsweise zur Herstellung einer wässrigen Lösung von Wasserstoffperoxid und/oder zur Herstellung einer wässrigen Lösung von hypochloriger Säure ausgestaltet sein. Die Herstellungsvorrichtung kann beispielsweise einen Elektrolyseur umfassen. Die Herstellungsvorrichtung erlaubt es vorteilhafterweise, Zusammensetzungen, die nicht oder nur mit hohem Aufwand lagerfähig sind, direkt vor ihrer Applikation herzustellen.

Das Gerät umfasst vorzugsweise einen Schallgenerator, bevorzugt einen Ultraschallgenerator, zur Beaufschlagung der ersten Zusammensetzung, der zweiten Zusammensetzung und/oder der dritten Zusammensetzung während der Applikation auf den Gewebeabschnitt und/oder des Spülfluids während der Spülung des Gewebeabschnitts mit Schall, bevorzugt mit Ultraschall. Durch die Beaufschlagung mit Schall, insbesondere mit Ultraschall, können die Zusammensetzungen und/oder das Spülfluid schneller und tiefer in den Gewebeabschnitt eindringen und so eine besonders effiziente und wirksame Spülung und/oder Behandlung bewirken.

Die Belichtungsvorrichtung umfasst vorzugsweise mindestens einen Laser, bevorzugt mindestens einen Diodenlaser, zur Erzeugung des Lichts. Ein Laser hat den Vorteil, dass er einen gebündelten Lichtstrahl mit hoher Intensität und definierter Wellenlänge bereitstellt. Ein Diodenlaser zeichnet sich durch eine geringe Größe, eine geringe Masse und einen geringen Energiebedarf aus. Der Diodenlaser kann beispielsweise in die Tragvorrichtung integriert sein. Die Belichtungsvorrichtung kann mindestens eine Glühlampe, insbesondere mindestens eine Halogen-Glühlampe, oder mindestens eine Leuchtdiode (LED) zur Erzeugung des Lichts umfassen.

Die Belichtungsvorrichtung ist vorzugsweise zur Belichtung mit Licht mit einer, bevorzugt einstellbaren, Wellenlänge in einem Bereich von 150 nm bis 1400 nm, bevorzugt von 400 nm bis 500 nm und/oder von 800 nm bis 1400 nm, besonders bevorzugt von 405 nm, 430 nm, 470 nm und/oder 490 nm, ausgelegt. Die genannten Bereiche und Wellenlängen führen, insbesondere mit Wasserstoffperoxid, Curcumin, Bengalrosa und/oder hypochloriger Säure in den Zusammensetzungen, zu einer besonders wirksamen Behandlung. Licht mit einer Wellenlänge im Bereich von 150 nm bis 600 nm kann vorzugsweise zur photodynamischen Desinfektion eingesetzt werden. Licht mit einer Wellenlänge im Bereich von 800 nm bis 1400 nm kann vorzugsweise zur Anregung der Wundheilung eingesetzt werden.

Die Belichtungsvorrichtung ist vorzugsweise zur Belichtung mit Licht mit einer, bevorzugt einstellbaren, Intensität in einem Bereich von 5 mW/cm² bis 500 mW/cm², bevorzugt von 50 mW/cm² bis 150 mW/cm², ausgelegt. Die genannten Bereiche führen, insbesondere mit Wasserstoffperoxid, Curcumin, Bengalrosa und/oder hypochloriger Säure in den Zusammensetzungen, zu einer besonders wirksamen Behandlung, ohne an den Gewebeabschnitt angrenzendes Gewebe zu beschädigen.

Eine Leistung des Lichts beträgt beispielsweise von 10 mW bis 500 mW, insbesondere von 50 mW bis 150 mW, bevorzugt 100 mW. Mit den genannten Leistungen lassen sich auf einer zur effizienten und präzisen Behandlung des Gewebeabschnitts geeigneten Belichtungsfläche in der Größenordnung von 1 cm² die vorgenannten vorteilhaften Intensitäten erreichen.

Die Belichtungsvorrichtung ist vorzugsweise zur Belichtung mit Licht mit einer vorprogrammierten Sequenz unterschiedlicher Wellenlängen ausgelegt. Durch eine vorprogrammierte Sequenz lassen sich automatisiert unterschiedliche Absorptionsbereiche eines oder mehrerer Photosensibilisatoren aktivieren, um eine besonders wirksame Behandlung, insbesondere gegen mehrere unterschiedliche Mikroorganismen, zu erreichen. Eine beispielhafte Sequenz, die insbesondere mit Wasserstoffperoxid, Bengalrosa, Curcumin und/oder hypochloriger Säure eine besonders wirksame Desinfektion zeigt, umfasst Belichtungen nacheinander mit Licht mit Wellenlängen von 405 nm, 430 nm, 470 nm und 490 nm für jeweils 10 min.

Die Belichtungsvorrichtung ist vorzugsweise zur Belichtung mit Licht während einer, beispielsweise auf 1 min bis 20 min, bevorzugt auf 2 min bis 15 min, besonders bevorzugt auf 3 min bis 10 min, vorprogrammierten Zeitdauer ausgelegt. Eine vorprogrammierte Zeitdauer erhöht die Sicherheit der Behandlung, da eine zu kurze Belichtung, die zu einer unvollständigen Desinfektion führen könnte, und eine zu lange Belichtung, die an den Gewebeabschnitt angrenzendes Gewebe schädigen könnte, vermieden werden. Die genannten Zeitdauern führen insbesondere mit Wasserstoffperoxid, Bengalrosa, Curcumin und/oder hypochloriger Säure zu einer wirksamen und sicheren Desinfektion.

Die Spülvorrichtung ist vorzugsweise zur Spülung des Gewebeabschnitts mit einem gasförmigen Spülfluid, bevorzugt mit Luft, und/oder mit einem flüssigen Spülfluid, bevorzugt mit einer wässrigen Lösung, ausgelegt. Die wässrige Lösung kann eine, bevorzugt physiologische, Kochsalzlösung sein.

Die Spülvorrichtung umfasst vorzugsweise einen Druckerzeuger, beispielsweise eine Pumpe, zur Beaufschlagung des Spülfluids mit einem, bevorzugt einstellbaren, Überdruck in einem Bereich von 1 bar bis 80 bar über einem Atmosphärendruck. Mit einem Überdruck in dem genannten Bereich kann der Gewebeabschnitt wirksam, schnell und für angrenzendes Gewebe schonend gespült werden. Ein einstellbarer Überdruck hat den Vorteil, dass er an die Art der Infektion und/oder des an den Gewebeabschnitt angrenzenden Gewebes angepasst werden kann.

Die Spülvorrichtung umfasst vorzugsweise ein das Spülfluid enthaltendes Vorratsgefäß. Damit kann das Gerät vorteilhafterweise unabhängig von einer externen Quelle für das Spülfluid betrieben werden.

Die Spülvorrichtung umfasst vorzugsweise eine Anschlussvorrichtung zum Anschluss der Spülvorrichtung an eine externe Versorgungsleitung zur Versorgung der Spülvorrichtung mit dem Spülfluid. Dadurch benötigt die Spülvorrichtung kein Vorratsgefäß für das Spülfluid und kann daher besonders platzsparend aufgebaut sein.

Die Absaugvorrichtung umfasst vorzugsweise eine Pumpe zum Absaugen des Spülfluids mit einem, bevorzugt einstellbaren, Unterdruck von 0,1 bar bis 0,8 bar, bevorzugt von 0,1 bar bis 0,3 bar, besonders bevorzugt von 0,1 bar bis 0,2 bar, unter einem Atmosphärendruck. Mit einem Unterdruck in dem genannten Bereich kann das Spülfluid von dem Gewebeabschnitt wirksam, schnell und für angrenzendes Gewebe schonend abgesaugt werden. Ein einstellbarer Unterdruck hat den Vorteil, dass er an die Art der Infektion und/oder des an den Gewebeabschnitt angrenzenden Gewebes angepasst werden kann.

Die Absaugvorrichtung umfasst vorzugsweise ein Entsorgungsgefäß zur Aufnahme des abgesaugten Spülfluids. Damit kann das Gerät vorteilhafterweise unabhängig von einer externen Senke für das Spülfluid betrieben werden.

Die Absaugvorrichtung umfasst vorzugsweise eine Anschlussvorrichtung zum Anschluss der Absaugvorrichtung an eine externe Entsorgungsleitung zur Entsorgung des abgesaugten Spülfluids. Dadurch benötigt die Absaugvorrichtung kein Entsorgungsgefäß für das Spülfluid und kann daher besonders platzsparend aufgebaut sein.

Das Gerät umfasst vorzugsweise eine, bevorzugt programmierbare, Steuervorrichtung zur, bevorzugt automatisierten, Steuerung der Spülvorrichtung, der Absaugvorrichtung, der Applikationsvorrichtung und/oder der Belichtungsvorrichtung. Die Steuervorrichtung kann das Gerät veranlassen, die einzelnen Schritte der Behandlung des Gewebeabschnitts automatisiert mit an die Art der Infektion und/oder des an den Gewebeabschnitt angrenzenden Gewebes angepassten Parametern durchzuführen, sodass die Behandlung einfacher und sicherer durchgeführt werden kann. Dabei kann eine manuelle Einstellung einzelner, mehrerer oder aller Parameter vor oder während der Behandlung möglich sein.

Die Steuervorrichtung umfasst beispielsweise ein kommunikativ mit der Spülvorrichtung, der Absaugvorrichtung, der Applikationsvorrichtung und/oder der Belichtungsvorrichtung verbundenes Computergerät. Die Steuervorrichtung umfasst vorzugsweise eine Benutzerschnittstelle, beispielsweise einen berührungsempfindlichen Bildschirm, zur Interaktion mit der Behandlungsperson.

Die Tragvorrichtung ist vorzugsweise als zur Durchführung der Behandlung von einer Behandlungsperson mit einer Hand zu haltendes Handstück ausgestaltet. Abmessungen, Form und Masse des Handstücks sind vorzugsweise so ausgestaltet, dass die Behandlungsperson die Behandlung mit dem Handstück ergonomisch durchführen kann.

Dadurch, dass die Applikationsöffnung und die Austrittsfläche sowie vorzugsweise die Spülöffnung und/oder die Absaugöffnung in das Handstück integriert sind, kann die Behandlungsperson mehrere oder sogar alle Schritte der Behandlung mit dem gleichen Handstück durchführen. Folglich ist die Behandlung besonders einfach und örtlich besonders exakt durchführbar.

Insbesondere wird durch die Integration in dem Handstück vermieden, dass die Zusammensetzungen außerhalb des Behandlungsbereichs appliziert werden, und dass eine Belichtung außerhalb des Behandlungsbereichs stattfindet. Dadurch wird eine besonders kleine Menge der Zusammensetzungen benötigt, und mögliche Nebenwirkungen werden vermieden.

Die Tragvorrichtung ist vorzugweise als Zelt zur Aufnahme des Menschen oder des Tieres ausgestaltet. Das Zelt kann dazu ausgestaltet sein, den Menschen oder das Tier vollständig oder teilweise aufzunehmen, beispielsweise kann das Zelt zur Aufnahme von einer Gliedmaße, von zwei Gliedmaßen, von einem Unterkörper und/oder von einem Oberkörper ausgestaltet sein. Das Zelt kann beispielsweise wie ein Saunazelt ausgestaltet sein. Die Ausgestaltung der Tragvorrichtung als Zelt eignet sich insbesondere zur effizienten Behandlung großflächiger Gewebeabschnitte.

In der Ausgestaltung der Tragvorrichtung als Zelt kann die Applikationsvorrichtung beispielsweise eine Tröpfchenbewässerung, eine Verdampfungsvorrichtung und/oder eine Vernebelungsvorrichtung für die Zusammensetzungen in dem Zelt umfassen. Dadurch können die Zusammensetzungen auch auf großflächige Gewebeabschnitte effizient appliziert werden.

In der Ausgestaltung der Tragvorrichtung als Zelt kann die Belichtungsvorrichtung beispielsweise ein LED-Band und/oder eine Anzahl von Lichtleitern zur großflächigen Belichtung der Zusammensetzungen umfassen.

Die Tragvorrichtung ist vorzugsweise als Atemmaske zur Beatmung des Menschen oder des Tieres ausgestaltet. Unter Atemmasken kommt es, insbesondere bei langandauernder Anwendung, häufig zu Infektionen und Entzündungen von Haut und Schleimhäuten. Wenn die Tragvorrichtung als Atemmaske ausgestaltet ist, ergibt sich daraus der Vorteil, dass eine Infektion oder Entzündung mit dem erfindungsgemäßen Gerät auf besonders einfache Weise behandelt werden kann, ohne die Beatmung des Menschen oder des Tieres zu unterbrechen.

Die Erfindung betrifft eine Kartusche zur Verwendung in einem erfindungsgemäßen Gerät. Die Applikationsvorrichtung des Geräts ist vorzugsweise zur austauschbaren Aufnahme der erfindungsgemäßen Kartusche ausgelegt. Die Erfindung betrifft auch ein erfindungsgemäßes Gerät umfassend eine erfindungsgemäße Kartusche, wobei die Kartusche vorzugsweise von der Applikationsvorrichtung des Geräts austauschbar aufgenommen ist.

Die Kartusche umfasst ein erstes Reservoir enthaltend die erste Zusammensetzung, vorzugsweise ein von dem ersten Reservoir flüssigkeitsdicht getrenntes zweites Reservoir enthaltend die zweite Zusammensetzung und besonders bevorzugt ein von dem ersten Reservoir und von dem zweiten Reservoir flüssigkeitsdicht getrenntes drittes Reservoir enthaltend die dritte Zusammensetzung. Dadurch wird eine Vermischung der Zusammensetzungen, die zu unerwünschten Wechselwirkungen, die die Wirksamkeit der Behandlung beeinträchtigen könnten, führen könnte, verhindert. Dennoch ist nur eine einzige Kartusche für die Zusammensetzungen notwendig, sodass das Beschaffung, Lagerung und Austausch der Kartusche einfacher sind, als wenn mehrere Kartuschen notwendig wären.

Die Kartusche umfasst vorzugsweise eine erste Entnahmeöffnung zur Entnahme der ersten Zusammensetzung aus dem ersten Reservoir, besonders bevorzugt eine zweite Entnahmeöffnung zur Entnahme der zweiten Zusammensetzung aus dem zweiten Reservoir und meist bevorzugt eine dritte Entnahmeöffnung zur Entnahme der dritten Zusammensetzung aus dem dritten Reservoir. Durch voneinander getrennte Entnahmeöffnungen wird eine unerwünschte Vermischung der Zusammensetzungen verhindert.

Vorzugsweise ist zumindest eine, insbesondere jeder, der Entnahmeöffnungen, durch ein Ventil, besonders bevorzugt durch ein Rückschlagventil, und/oder durch ein Septum verschlossen. Ein Rückschlagventil hat den Vorteil, dass es ein Eindringen von Fremdstoffen in das zugehörige Reservoir verhindert. Ein Septum hat die Vorteile, dass es die zugehörige Entnahmeöffnung zuverlässig verschließt und auf einfache Weise, beispielsweise mit einer Kanüle der Applikationsvorrichtung des Geräts, geöffnet werden kann.

Vorzugsweise ist zumindest eines, insbesondere jedes, der Reservoire der Kartusche lichtdicht abgeschlossen. Dadurch können auch Zusammensetzungen, die lichtempfindliche Komponenten enthalten, ohne zusätzliche Schutzmaßnahmen in dem Reservoir gelagert werden.

Zumindest eine, insbesondere jede, der Zusammensetzungen weist in der Kartusche einen Überdruck über einem Atmosphärendruck auf. Dadurch kann die Zusammensetzung, sobald die Kartusche geöffnet wird, von dem Überdruck angetrieben aus der Kartusche austreten und auf den Gewebeabschnitt appliziert werden, ohne dass dazu ein zusätzlicher Antrieb, beispielsweise eine Pumpe, notwendig wäre. Das Gerät kann also besonders einfach aufgebaut sein.

Die erste von dem Gerät applizierbare und/oder in der Kartusche enthaltene Zusammensetzung enthält vorzugsweise Curcumin, Bengalrosa, 5-Aminolävulinsäure, Embelin und/oder Methylenblau. Diese Photosensibilisatoren, insbesondere eine Kombination von Curcumin mit Bengalrosa, erlauben eine besonders wirksame Desinfektion des Gewebeabschnitts.

Die erste von dem Gerät applizierbare und/oder in der Kartusche enthaltene Zusammensetzung enthält bevorzugt Chitosan-Nanopartikel. Durch Zugabe von Chitosan-Nanopartikeln kann die Wirksamkeit der Photosensibilisatoren, insbesondere der vorgenannten Photosensibilisatoren, verstärkt werden.

Die zweite von dem Gerät applizierbare und/oder in der Kartusche enthaltene Zusammensetzung enthält oder ist vorzugsweise hypochlorige Säure, bevorzugt als wässrige Lösung mit einem Stoffmengenanteil von 0,01 % bis 1 %, besonders bevorzugt von 0,04 % bis 0,5 %. Eine zweistufige Desinfektion mit einer solchen zweiten Zusammensetzung und einer wie zuvor beschrieben ausgestalteten ersten Zusammensetzung, insbesondere mit Curcumin und Bengalrosa, ist besonders wirksam.

Die hypochlorige Säure enthält vorzugsweise kein Natriumhypochlorid, da dieses gefäßschädigend wirkt. Hypochlorige Säure ist wegen ihrer Wirkung als Oxidationsmittel und wegen ihres niedrigen pH-Werts mikrobiell nicht anfällig.

Die dritte von dem Gerät applizierbare und/oder in der Kartusche enthaltene Zusammensetzung enthält oder ist beispielsweise eine wässrige Lösung von Chlorhexidin, insbesondere mit einer Chlorhexidin-Konzentration von 0,05 % bis 0,25 %, vorzugsweise von 0,1 % bis 0,2 %, besonders bevorzugt von 0,12 %.

Die dritte von dem Gerät applizierbare und/oder in der Kartusche enthaltene Zusammensetzung enthält oder ist beispielsweise eine wässrige Lösung von Wasserstoffperoxid, insbesondere mit einer WasserstoffperoxidKonzentration von 0,5 % bis 10 %, vorzugsweise von 1 % bis 5 %, besonders bevorzugt von 1,5 % bis 3,0 %. Chlorhexidin und Wasserstoffperoxid in den genannten Konzentrationen zeigen jeweils eine wirksame Wunddesinfektion, die durch Belichtung verstärkt werden kann.

Die Kartusche umfasst vorzugsweise eine Temperaturanzeige zur Anzeige einer Überschreitung einer maximalen Lagertemperatur der Kartusche und/oder zur Anzeige einer Unterschreitung einer minimalen Lagertemperatur der Kartusche. Mit der Temperaturanzeige kann eine Person, die die Kartusche verwendet, auf einfache Weise erkennen, ob diese außerhalb des vorgesehenen Temperaturbereichs gelagert wurde, wodurch die Wirksamkeit der Zusammensetzungen beeinträchtigt sein könnte. Somit wird die Sicherheit der Verwendung der Kartusche erhöht.

Die Temperaturanzeige kann beispielsweise zumindest einen Farbstoff enthalten, der seine Farbe bei Überschreitung der maximalen Lagertemperatur oder Unterschreitung der minimalen Lagertemperatur ändert.

Die Kartusche ist vorzugsweise im Kühlschrank aufzubewahren und darf im Zusammenhang mit der Verwendung nicht unnötig lange unverschlossen stehen gelassen oder dem Licht ausgesetzt werden, um die Stabilität und Keimfreiheit der Zusammensetzungen zu gewährleisten. Der Temperatureffekt auf die Stabilität kann deutlich sein, bereits zwischen 20 °C und 25 °C verdoppelt sich beispielsweise die Zersetzungsgeschwindigkeit von hypochloriger Säure.

Die Erfindung betrifft eine Zusammensetzung zur Behandlung eines Gewebeabschnitts, insbesondere einer Wunde, eines Menschen oder eines Tieres mit einem erfindungsgemäßen Gerät. Die Erfindung betrifft auch ein erfindungsgemäßes Gerät und eine erfindungsgemäße Kartusche, die jeweils die erfindungsgemäße Zusammensetzung als erste Zusammensetzung umfassen.

Die Zusammensetzung ist gelförmig. Sie ist also ein disperses System, das eine festen und eine flüssige Komponente umfasst. Die feste Komponente bildet dabei eine schwammartige, dreidimensionale Matrix, deren Poren durch eine flüssige Komponente ausgefüllt sind. Die flüssige Komponente ist dadurch in der festen Komponente immobilisiert. Somit verbleibt die gelförmige Zusammensetzung im Gegensatz zu einer flüssigen Zusammensetzung dort, wo sie auf den Gewebeabschnitt appliziert wird. Dadurch wird eine besonders kleine Menge der Zusammensetzung benötigt, und mögliche Nebenwirkungen durch eine sich unkontrolliert verbreitende Zusammensetzung werden vermieden. Ein Kontakt einer, insbesondere erfindungsgemäßen, Zusammensetzung mit Schleimhäuten oder offenen Wunden könnte beispielsweise zu Irritationen führen.

Die erfindungsgemäße Zusammensetzung umfasst Curcumin mit einem Massenanteil von 0,1 % bis 10 %, bevorzugt von 0,5 % bis 5 %, an der Zusammensetzung. Die gelförmige Zusammensetzung umfasst Bengalrosa mit einem Massenanteil von 0,01 % bis 1 %, bevorzugt von 0,05 % bis 0,5 %, an der Zusammensetzung. Die Kombination von Curcumin und Bengalrosa in den genannten Anteilen führt zu einer besonders wirksamen Desinfektion des Gewebeabschnitts.

Die erfindungsgemäße Zusammensetzung umfasst eine Gelbildner-Zusammensetzung und Wasser. Die Gelbildner-Zusammensetzung bildet die feste Matrix der gelförmigen Zusammensetzung. Wasser dient als zur Wundbehandlung unbedenkliche Trägersubstanz für die übrigen Komponenten der gelförmigen Zusammensetzung.

Die Gelbildner-Zusammensetzung enthält vorzugsweise ein Poloxamer, beispielsweise Poloxamer 407, mit einem Massenanteil von 0,2 % bis 20 %, bevorzugt von 1 % bis 10 %, an der Zusammensetzung. Poloxamere sind Blockcopolymere aus Ethylenoxid und Propylenoxid. Es handelt sich um schaumarme und schaumdämpfende, nichtionische Tenside. Poloxamer 407 hat einen relativen Massenanteil der Ethylenoxideinheiten von 70 %, eine relative Molekülmasse des Polypropylenglycol-Blocks von 4000 und eine gesamte mittlere relative Molekülmasse von 9840 bis 14600.

Die Gelbildner-Zusammensetzung enthält vorzugsweise 1,2-Propylenglycol mit einem Massenanteil von 0,13 % bis 13 %, bevorzugt von 0,63 % bis 6,3 %, an der Zusammensetzung. 1,2-Propylenglycol, auch bekannt als 1,2-Propandiol, ist eine klare, farblose, nahezu geruchlose und stark hygroskopische Flüssigkeit. 1,2-Propylenglycol ist ein gesundheitlich unbedenkliches Lösungsmittel und hat antimikrobielle Eigenschaften, sodass auf zusätzliche Konservierungsstoffe verzichtet werden kann.

Die Gelbildner-Zusammensetzung enthält vorzugsweise 2-Propanol mit einem Massenanteil von 0,13 % bis 13 %, bevorzugt von 0,63 % bis 6,3 %, an der Zusammensetzung. 2-Propanol (IUPAC-Name Propan-2-ol), auch als Isopropylalkohol oder Isopropanol (abgekürzt IPA) bekannt, ist der einfachste nicht cyclische, sekundäre Alkohol und ein einwertiger Alkohol. 2-Propanol ist ein Lösungsmittel mit geringer Toxizität und hat antimikrobielle Eigenschaften, sodass auf zusätzliche Konservierungsstoffe verzichtet werden kann.

Die Gelbildner-Zusammensetzung enthält vorzugsweise mittelkettige Triglyceride mit einem Massenanteil von 0,05 % bis 5 %, bevorzugt von 0,25 % bis 2,5 %, an der Zusammensetzung. Triglyceride, auch Glycerol-Triester, sind dreifache Ester des dreiwertigen Alkohols Glycerin mit drei Fettsäuremolekülen und sollten nach der IUPAC-Empfehlung als Triacylglycerole bezeichnet werden. Mittelkettige Triglyceride haben Fettsäuren mittlerer Länge (6 bis 12 C-Atome).

Die erfindungsgemäße Zusammensetzung ist ab ihrer Herstellung vorzugsweise für mindestens 6 Monate, besonders bevorzugt für mindestens 12 Monate haltbar, insbesondere wenn die Zusammensetzung bei Kühlschranktemperatur gelagert wird.

Die vorliegende Offenbarung betrifft auch ein Verfahren zur Herstellung einer erfindungsgemäßen Zusammensetzung.

Das Verfahren umfasst ein Vermischen des Curcumins, des Bengalrosas, des Poloxamers, des 1,2-Propylenglycols, des 2-Propanols und des Wassers zu einer Mischung. Das Vermischen erfolgt beispielsweise mit einem Stößel oder mit einem Pistill in einem Mörser, insbesondere in einer Fantaschale.

Das Verfahren umfasst ein Ruhenlassen der Mischung während einer Ruhedauer von mindestens 30 min, bevorzugt mindestens 45 min, besonders bevorzugt mindestens 60 min. Das Ruhenlassen führt dazu, dass sich beim Vermischen entstandene Luftblasen und/oder Gelklumpen auflösen können.

Während des Ruhenlassens wird die Mischung vorzugsweise abgedeckt, um Kontaminationen zu vermeiden, und kann gelegentlich umgerührt werden, um die Auflösung von Gelklumpen und/oder Luftblase zu unterstützen.

Das Verfahren umfasst ein Einmischen der mittelkettigen Triglyceride in die Mischung nach dem Ruhenlassen. Durch das Einmischen entsteht ein homogenes Gel.

Während des Vermischens, Ruhenlassens und/oder Einmischens aufgetretene Verdunstungsverluste können durch Einrühren von 2-Propanol in die Zusammensetzung ausgeglichen werden.

Das Verfahren kann ein Einstellen eines pH-Werts der Zusammensetzung auf einen leicht sauren pH-Wert von vorzugsweise 6,0 bis 6,7 umfassen. Ein leicht saurer pH-Wert vermindert die Vermehrungsfähigkeit von häufig in Wundinfektionen anzutreffenden Bakterien, beispielsweise von *Propionibacterium acnes.*

Das Verfahren kann ein Abfüllen der Zusammensetzung in ein Gefäß, insbesondere in eine erfindungsgemäße Kartusche, umfassen, wobei das Gefäß nach dem Abfüllen dicht verschlossen wird, um Kontaminationen zu vermeiden.

Die Zusammensetzung wird vorzugsweise bei Kühlschranktemperatur, beispielsweise bei einer Temperatur von 3 °C bis 9 °C, gelagert, um ein Wachstum von Mikroorganismen zu verringern.

### Beispiele

Die erfindungsgemäße Zusammensetzung besteht beispielsweise aus folgenden Inhaltsstoffen pro 100 g der Zusammensetzung:
a. 10,0 g Curcumin,
b. 1,0 g Bengalrosa,
c. 20,0 g Poloxamer 407,
d. 12,5 g Propylenglykol,
e. 12,5 g 2-Propanol,
f. 5,0 g mittelkettige Triglyceride und
g. 39,0 g Wasser.

Das Verfahren zur Herstellung einer erfindungsgemäßen Zusammensetzung, beispielsweise aus den im vorherigen Absatz genannten Inhaltsstoffen, umfasst beispielsweise folgende Schritte:
a. In einer mit Pistill tarierten Fantaschale werden Curcumin, Bengalrosa, Poloxamer mit Propylenglycol, gereinigtem Wasser und 2-Propanol vorsichtig vermischt. Luftblasen und Gelklumpen in der Mischung sind normal in diesem Herstellungsschritt.
b. Die Mischung wird abgedeckt mindestens 60 min unter gelegentlichem Umrühren ruhen gelassen. Dabei ist die Mischung nach 60 min klar und viskos und zeigt wenige Luftblasen.
c. Die mittelkettigen Trigyceride werden vorsichtig in die Mischung eingemischt. Dabei wird die Mischung weiß und viskos und zeigt wenige Luftblasen.
d. Verdunstungsverluste werden mit 2-Propanol ausgeglichen und verrührt.
e. Die fertige gelförmige Zusammensetzung muss weiß und homogen aussehen und nach 2-Propanol riechen und darf keine Luftblasen enthalten.
f. Der pH-Wert soll bei 6,0 bis 6,7 liegen. Da sich Bakterien, beispielsweise *Propionibacterium acnes,* ab einen pH-Wert von 6,8 vermehren, wird der pH-Wert gegebenenfalls auf unter 6,8 eingestellt.
g. Die gelförmige Zusammensetzung wird direkt abgefüllt und dicht verschlossen.

Eine mit dem erfindungsgemäßen Gerät durchgeführte Behandlung einer Wunde umfasst beispielsweise folgende Schritte, die beispielsweise zweimal pro Woche über vier Wochen wiederholt werden:
a. Spülen der Wunde mit dem Spülfluid zur Entfernung von beschädigtem Gewebe und/oder Fremdkörpern,
b. Applizieren der ersten, insbesondere erfindungsgemäßen, Zusammensetzung, beispielsweise als dünner Film mit einer Belegdichte von 0,05 -0,2 g/cm², in die Wunde,
c. Belichten der ersten Zusammensetzung mindestens mit Wellenlängen von 405 nm, 430 nm, 470 nm und 490 nm für jeweils 10 min,
d. Abwischen der ersten Zusammensetzung nach dem Belichten, beispielsweise mit einem milden Gesichtswasser,
e. Applizieren der zweiten Zusammensetzung, insbesondere einer wässrigen Lösung von hypochloriger Säure mit einem Stoffmengenanteil von 400 ppm, wobei gegebenenfalls reaktive Sauerstoffspezies mit Zitronensäure entfernt werden können,
f. Belichten der zweiten Zusammensetzung mindestens mit Wellenlängen von 405 nm, 430 nm, 470 nm und 490 nm für jeweils 10 min.

Eine weitere mit dem erfindungsgemäßen Gerät durchgeführte Behandlung einer Wunde umfasst beispielsweise folgende Schritte:
a. Applizieren einer Zusammensetzung, insbesondere einer wässrigen Lösung von Wasserstoffperoxid mit einem Massenanteil von 1,5 % oder 3,0 % oder einer wässrigen Lösung von Chlorhexidin mit einem Massenanteil von 0,12 %, in die Wunde,
b. Belichten der Zusammensetzung mit Licht mit einer Wellenlänge von 405 nm während einer Belichtungsdauer von 3 min.

### Kurze Beschreibung der Zeichnungen

Weitere Vorteile, Ziele und Eigenschaften der Erfindung werden anhand nachfolgender Beschreibung und anliegender Zeichnungen erläutert, in welchen beispielhaft erfindungsgemäße Gegenstände dargestellt sind.
Figur 1 zeigt eine schematische Darstellung eines erfindungsgemäßen Gerätes.
Figur 2 zeigt eine schematische Darstellung einer erfindungsgemäßen Kartusche.

### Fig.1

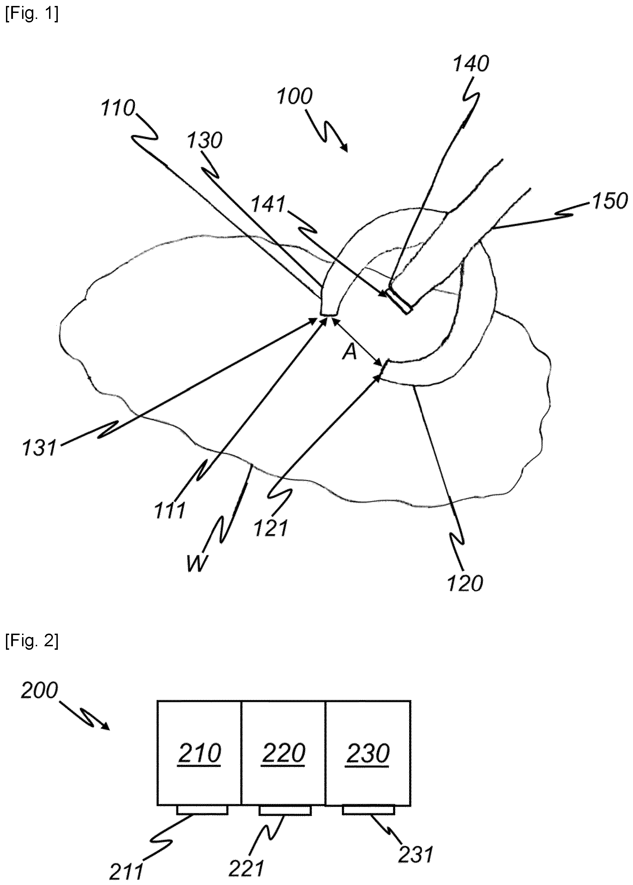

Figur 1 zeigt eine schematische Darstellung eines erfindungsgemäßen Gerätes 100 zur Behandlung eines Gewebeabschnitts W, insbesondere einer Wunde, eines Menschen oder eines Tieres.

Das gezeigte Gerät 100 umfasst eine Spülvorrichtung 110 zur Spülung des Gewebeabschnitts W mit einem Spülfluid, wobei die Spülvorrichtung 110 eine Spülöffnung 111 zur Abgabe des Spülfluids aus der Spülvorrichtung 110 an den Gewebeabschnitt W umfasst.

Das gezeigte Gerät 100 umfasst eine Absaugvorrichtung 120 zur Absaugung des Spülfluids von dem Gewebeabschnitt W, wobei die Absaugvorrichtung 120 eine Absaugöffnung 121 zur Aufnahme des Spülfluids von dem Gewebeabschnitt W in die Absaugvorrichtung 120 umfasst.

Das gezeigte Gerät 100 umfasst eine Applikationsvorrichtung 130 zur Applikation einer ersten Zusammensetzung, einer zweiten Zusammensetzung und einer dritten Zusammensetzung zur Behandlung des Gewebeabschnitts W auf den Gewebeabschnitt W, wobei die Applikationsvorrichtung 130 eine Applikationsöffnung 131 zur Abgabe der Zusammensetzungen aus der Applikationsvorrichtung 130 an den Gewebeabschnitt W umfasst.

Die Applikationsöffnung 131 ist beispielsweise nahe der Spülöffnung 111 angeordnet. Die Applikationsöffnung 131 kann mit der Spülöffnung 111 identisch sein.

Das gezeigte Gerät 100 umfasst eine Belichtungsvorrichtung 140 zur Belichtung der auf den Gewebeabschnitt W applizierten Zusammensetzungen mit Licht, wobei die Belichtungsvorrichtung 140 eine Austrittsfläche 141 zum Austritt des Lichts aus der Belichtungsvorrichtung 140 umfasst. Die Belichtungsvorrichtung 140 umfasst beispielsweise einen Diodenlaser zur Erzeugung des Lichts.

Das gezeigte Gerät 100 umfasst eine als Handstück ausgestaltete Tragvorrichtung 150, wobei die Spülöffnung 111, die Absaugöffnung 121, die Applikationsöffnung 131 und die Austrittsfläche 141 in das Handstück 150 integriert sind.

Ein Abstand A der Spülöffnung 111 von der Absaugöffnung 121 beträgt beispielsweise von 1 mm bis 5 mm.

### Fig.2

Figur 2 zeigt eine schematische Darstellung einer erfindungsgemäßen Kartusche 200 zur Verwendung in einem erfindungsgemäßen Gerät 100.

Die gezeigte Kartusche (200) umfasst ein erstes Reservoir (210) enthaltend die erste Zusammensetzung, beispielsweise eine erfindungsgemäße Zusammensetzung, und ein von dem ersten Reservoir (210) flüssigkeitsdicht getrenntes zweites Reservoir (220) enthaltend die zweite Zusammensetzung, beispielsweise eine wässrige Lösung von hypochloriger Säure. Die Kartusche (200) umfasst ein von dem ersten Reservoir 210 und von dem zweiten Reservoir 220 flüssigkeitsdicht getrenntes drittes Reservoir 230 enthaltend die dritte Zusammensetzung, beispielsweise eine wässrige Lösung von Wasserstoffperoxid.

Die gezeigte Kartusche weist eine erste Entnahmeöffnung (211) zur Entnahme der ersten Zusammensetzung aus dem ersten Reservoir (210), eine zweite Entnahmeöffnung (221) zur Entnahme der zweiten Zusammensetzung aus dem zweiten Reservoir (220) und eine dritte Entnahmeöffnung 231 zur Entnahme der dritten Zusammensetzung aus dem dritten Reservoir 230 auf.

### Liste der Bezugszeichen

| | | | |
|---|---|---|---|
| 100 | Gerät | 150 | Tragvorrichtung |
| 110 | Spülvorrichtung | 200 | Kartusche |
| 111 | Spülöffnung | 210 | erstes Reservoir |
| 120 | Absaugvorrichtung | 211 | erste Entnahmeöffnung |
| 121 | Absaugöffnung | 220 | zweites Reservoir |
| 130 | Applikationsvorrichtung | 221 | zweite Entnahmeöffnung |
| 131 | Applikationsöffnung | 230 | drittes Reservoir |
| 140 | Belichtungsvorrichtung | 231 | dritte Entnahmeöffnung |
| 141 | Austrittsfläche | A | Abstand |
| | | W | Gewebeabschnitt |

## Patentansprüche

1. Gerät (100) zur Behandlung eines Gewebeabschnitts (W), insbesondere einer Wunde, eines Menschen oder eines Tieres, das Gerät (100) umfassend
a. eine Applikationsvorrichtung (130) zur Applikation einer ersten Zusammensetzung, einer zweiten Zusammensetzung und einer dritten Zusammensetzung zur Behandlung des Gewebeabschnitts (W) auf den Gewebeabschnitt (W), wobei die Applikationsvorrichtung (130) mindestens eine Applikationsöffnung (131) zur Abgabe der Zusammensetzungen aus der Applikationsvorrichtung (130) an den Gewebeabschnitt (W) umfasst, und
b. eine Belichtungsvorrichtung (140) zur Belichtung der auf den Gewebeabschnitt (W) applizierten Zusammensetzungen mit Licht, wobei die Belichtungsvorrichtung (140) eine Austrittsfläche (141) zum Austritt des Lichts aus der Belichtungsvorrichtung (140) umfasst,
c. eine Tragvorrichtung (150), wobei die Tragvorrichtung (150) die mindestens eine Applikationsöffnung (131) und die Austrittsfläche (141) trägt, und
d. einen Schallgenerator zur Beaufschlagung der ersten Zusammensetzung, der zweiten Zusammensetzung und/oder der dritten Zusammensetzung während der Applikation auf den Gewebeabschnitt (W) und/oder des Spülfluids während der Spülung des Gewebeabschnitts (W) mit Schall,
e. wobei die Belichtungsvorrichtung (140) zur Belichtung mit Licht mit einer Wellenlänge in einem Bereich von 400 nm bis 500 nm und in einem Bereich von 800 nm bis 1400 nm ausgelegt ist,
**dadurch gekennzeichnet, dass**
f. das Gerät (100) eine Spülvorrichtung (110) zur Spülung des Gewebeabschnitts (W) mit einem Spülfluid umfasst, wobei die Spülvorrichtung (110) mindestens eine von der Tragvorrichtung (150) getragene Spülöffnung (111) zur Abgabe des Spülfluids aus der Spülvorrichtung (110) an den Gewebeabschnitt (W) umfasst,
g. wobei die Spülvorrichtung (110) einen Druckerzeuger zur Beaufschlagung des Spülfluids mit einem Überdruck in einem Bereich von 1 bar bis 80 bar über einem Atmosphärendruck umfasst,
h. wobei von der Applikationsvorrichtung (130) mindestens eine Kartusche (200) enthaltend zumindest die zweite Zusammensetzung und die dritte Zusammensetzung austauschbar aufgenommen ist,
i. wobei die zweite Zusammensetzung eine wässrige Lösung von hypochloriger Säure ist, und
j. wobei die dritte Zusammensetzung eine wässrige Lösung von Wasserstoffperoxid ist.

2. Gerät (100) nach Anspruch 1,
**dadurch gekennzeichnet, dass**
das Gerät (100) eine Absaugvorrichtung (120) zur Absaugung des Spülfluids von dem Gewebeabschnitt (W) umfasst, wobei die Absaugvorrichtung (120) mindestens eine von der Tragvorrichtung (150) getragene Absaugöffnung (121) zur Aufnahme des Spülfluids von dem Gewebeabschnitt (W) in die Absaugvorrichtung (120) umfasst.

3. Gerät (100) nach Anspruch 1,
**dadurch gekennzeichnet, dass**
die Applikationsvorrichtung (130) eine Fluss-Steuereinheit zur Steuerung jeweils eines Flusses der drei Zusammensetzungen auf den Gewebeabschnitt (W) umfasst.

4. Gerät (100) nach Anspruch 1,
**dadurch gekennzeichnet, dass**
die Applikationsvorrichtung (130) eine Zerstäubungseinheit zur Zerstäubung der ersten Zusammensetzung, der zweiten Zusammensetzung und/oder der dritten Zusammensetzung bei der Applikation auf den Gewebeabschnitt (W) umfasst.

5. Gerät (100) nach Anspruch 1,
**dadurch gekennzeichnet, dass**
die Belichtungsvorrichtung (140) zur Belichtung mit Licht mit einer Intensität in einem Bereich von 50 mW/cm² bis 150 mW/cm² ausgelegt ist.

6. Gerät (100) nach Anspruch 1,
**dadurch gekennzeichnet, dass**
die Tragvorrichtung (150) als zur Behandlung des Gewebeabschnitts (W) von einer Behandlungsperson mit einer Hand zu haltendes Handstück ausgestaltet ist.

7. Gerät (100) nach Anspruch 1,
**dadurch gekennzeichnet, dass**
die Tragvorrichtung (150) als Zelt zur Aufnahme des Menschen oder des Tieres ausgestaltet ist.

8. Gerät (100) nach Anspruch 1,
**dadurch gekennzeichnet, dass**
die Tragvorrichtung (150) als Atemmaske zur Beatmung des Menschen oder des Tieres ausgestaltet ist.

9. Gerät (100) nach Anspruch 1, die Kartusche (200) umfassend
a. ein erstes Reservoir (210) enthaltend die erste Zusammensetzung,
b. ein von dem ersten Reservoir (210) flüssigkeitsdicht getrenntes zweites Reservoir (220) enthaltend die zweite Zusammensetzung und
c. ein von dem ersten Reservoir (210) und von dem zweiten Reservoir (220) flüssigkeitsdicht getrenntes drittes Reservoir (230) enthaltend die dritte Zusammensetzung.

10. Gerät (100) nach Anspruch 1,
**dadurch gekennzeichnet, dass**
die Kartusche (200) eine erste Entnahmeöffnung (211) zur Entnahme der ersten Zusammensetzung aus dem ersten Reservoir (210), eine zweite Entnahmeöffnung (221) zur Entnahme der zweiten Zusammensetzung aus dem zweiten Reservoir (220) und eine dritte Entnahmeöffnung (231) zur Entnahme der dritten Zusammensetzung aus dem dritten Reservoir (230) aufweist, wobei zumindest eine der Entnahmeöffnungen (211, 221, 231) durch ein Ventil verschlossen ist.

11. Gerät (100) nach Anspruch 1 umfassend die erste Zusammensetzung, wobei die erste Zusammensetzung gelförmig ist und folgende Komponenten umfasst:
a. Curcumin mit einem Massenanteil von 0,1 % bis 10 % an der ersten Zusammensetzung,
b. Bengalrosa mit einem Massenanteil von 0,01 % bis 1 % an der ersten Zusammensetzung,
c. eine Gelbildner-Zusammensetzung und
d. Wasser.

12. Gerät (100) nach Anspruch 11,
**dadurch gekennzeichnet, dass**
die Gelbildner-Zusammensetzung
a. ein Poloxamer mit einem Massenanteil von 0,2 % bis 20 % an der ersten Zusammensetzung enthält,
b. 1,2-Propylenglycol mit einem Massenanteil von 0,13 % bis 13 % an der ersten Zusammensetzung enthält,
c. 2-Propanol mit einem Massenanteil von 0,13 % bis 13 % an der ersten Zusammensetzung enthält, und/oder
d. mittelkettige Triglyceride mit einem Massenanteil von 0,05 % bis 5 % an der ersten Zusammensetzung enthält.

## Claims

1. A device (100) for treating a tissue portion (W), in particular a wound, of a human or of an animal, the device (100) comprising
a. an application device (130) for applying a first composition, a second composition, and a third composition for treating the tissue section (W) onto the tissue section (W), wherein the application device (130) comprises at least one application opening (131) for dispensing the compositions from the application device (130) to the tissue section (W), and
b. an exposure device (140) for exposing the compositions applied to the tissue section (W) with light, the exposure device (140) having an exit surface (141) for exiting the light from the exposure device (140),
c. a carrying device (150), wherein the carrying device (150) carries the at least one application opening (131) and the exit surface (141), and
d. a sound generator for exposing the first composition, the second composition, and/or the third composition during application to the tissue section (W) and/or a rinsing fluid during the rinsing of the tissue section (W) to sound,
e. wherein the exposure device (140) is designed for emitting light having a wavelength in a range from 400 nm to 500 nm and in a range from 800 nm to 1400 nm,
**characterized in that**
f. the device (100) comprises a rinsing device (110) for rinsing the tissue section (W) with the rinsing fluid, wherein the rinsing device (110) comprises at least one rinsing opening (111) carried by the carrying device (150) for dispensing the rinsing fluid from the rinsing device (110) to the tissue section (W),
g. wherein the rinsing device (110) comprises a pressure generator for supplying the rinsing fluid with an overpressure in a range from 1 bar to 80 bar above an atmospheric pressure,
h. wherein the application device (130) contains at least one exchangeable cartridge (200) containing at least the second composition and the third composition,
i. wherein the second composition is an aqueous solution of hypochlorous acid, and
j. wherein the third composition is an aqueous solution of hydrogen peroxide.

2. The device (100) according to claim 1,
**characterized in that**
the device (100) comprises a suction device (120) for extracting the rinsing fluid from the tissue section (W), wherein the suction device (120) comprises at least one suction opening (121) carried by the carrying device (150) for receiving the rinsing fluid from the tissue section (W) into the suction device (120).

3. The device (100) according to claim 1,
**characterized in that**
the application device (130) comprises a flow control unit for controlling a flow of each of the three compositions onto the tissue section (W).

4. The device (100) according to claim 1,
**characterized in that**
the application device (130) comprises an atomizing unit for atomizing the first composition, the second composition, and/or the third composition upon application to the tissue section (W).

5. The device (100) according to claim 1,
**characterized in that**
the exposure device (140) is designed for emitting light having an intensity in a range of 50 mW/cm² to 150 mW/cm².

6. The device (100) according to claim 1,
**characterized in that**
the carrying device (150) is configured as a hand piece to be held by a practitioner with a hand for treating the tissue section (W).

7. The device (100) according to claims 1,
**characterized in that**
the carrying device (150) is configured as a tent for receiving the human or the animal.

8. The device (100) according to claims 1,
**characterized in that**
the carrying device (150) is configured as a breathing mask for respiration of the human or of the animal.

9. The device (100) according to claim 1, the cartridge (200) comprising
a. a first reservoir (210) containing the first composition;
b. a second reservoir (220), separated from the first reservoir (210) in a liquid-tight manner and containing the second composition; and
c. a third reservoir (230), separated from the first reservoir (210) and from the second reservoir (220) in a liquid-tight manner and containing the third composition.

10. The device (100) according to claim 9,
**characterized in that**
the cartridge (200) comprises a first removal opening (211) for removing the first composition from the first reservoir (210), a second removal opening (221) for removing the second composition from the second reservoir (220), and a third removal opening (231) for removing the third composition from the third reservoir (230), wherein at least one of the removal openings (211, 221, 231) is closed by a valve.

11. The device (100) according to claim 1 comprising the first composition, wherein the first composition is gel-like and comprises the following components:
a. curcumin with a mass fraction of from 0.1 % to 10 % of the first composition,
b. rose bengal having a mass fraction of from 0.01 % to 1 % of the first composition,
c. a gel-forming composition, and
d. water.

12. The device (100) of claim 11,
**characterized in that**
the gel-forming composition comprises
a. a poloxamer having a mass fraction of from 0.2 % to 20 % of the first composition,
b. 1, 2-propylene glycol having a mass fraction of from 0.13 % to 13 % of the first composition,
c. 2-propanol having a mass fraction of from 0.13 % to 13 % of the first composition, and/or
d. medium chain-length triglycerides having a mass fraction of from 0.05 % to 5 % of the first composition.

## Revendications

1. Dispositif (100) pour le traitement d'une région de tissu (W), en particulier une blessure, d'un être humain ou d'un animal, le dispositif (100) comprenant
a. un dispositif d'application (130) pour appliquer une première composition, une deuxième composition et une troisième composition pour traiter la région de tissu (W) sur la région de tissu (W), le dispositif d'application (130) comprenant au moins un orifice d'application (131) pour délivrer les compositions depuis le dispositif d'application (130) à la région de tissu (W), et
b. un dispositif d'exposition (140) pour exposer à la lumière les compositions appliquées sur la région de tissu (W), le dispositif d'exposition (140) comprenant une surface de sortie (141) pour faire sortir la lumière du dispositif d'exposition (140),
c. un dispositif de support (150), ledit dispositif de support (150) portant ledit au moins un orifice d'application (131) et ladite surface de sortie (141), et
d. un générateur de son pour exposer au son la première composition, la deuxième composition et/ou la troisième composition pendant l'application sur la région de tissu (W) et/ou un fluide de rinçage pendant le rinçage de la région de tissu (W),
e. le dispositif d'exposition (140) étant adapté pour émettre une lumière ayant une longueur d'onde dans une plage de 400 nm à 500 nm et dans une plage de 800 nm à 1400 nm,
**caractérisé en ce que**
f. le dispositif (100) comprend un dispositif de rinçage (110) pour irriguer la région de tissu (W) avec le fluide de rinçage, le dispositif de rinçage (110) comprenant au moins un orifice de rinçage (111) porté par le dispositif de support (150) pour distribuer le fluide de rinçage du dispositif de rinçage (110) à la région de tissu (W),
g. le dispositif de rinçage (110) comprenant un générateur de pression pour appliquer au fluide de rinçage une pression positive dans une plage de 1 bar à 80 bars au-dessus d'une pression atmosphérique,
h. le dispositif d'application (130) contenant au moins une cartouche (200) interchangeable contenant au moins la deuxième composition et la troisième composition,
i. la deuxième composition étant une solution aqueuse d'acide hypochloreux, et
j. la troisième composition étant une solution aqueuse de peroxyde d'hydrogène.

2. Dispositif (100) selon la revendication 1,
**caractérisé en ce que**
le dispositif (100) comprend un dispositif d'aspiration (120) pour aspirer le fluide de rinçage de la région de tissu (W), le dispositif d'aspiration (120) comprenant au moins une ouverture d'aspiration (121) portée par le dispositif de support (150) pour recevoir le fluide de rinçage de la région de tissu (W) dans le dispositif d'aspiration (120).

3. Dispositif (100) selon la revendication 1,
**caractérisé en ce que**
le dispositif d'application (130) comprend une unité de réglage de flux pour régler un flux de chacune des trois compositions sur la région de tissu (W).

4. Dispositif (100) selon la revendication 1,
**caractérisé en ce que**
le dispositif d'application (130) comprend une unité d'atomisation pour atomiser la première composition, la deuxième composition et/ou la troisième composition lors de l'application sur la région de tissu (W).

5. Dispositif (100) selon la revendication 1,
**caractérisé en ce que**
le dispositif d'exposition (140) est conçu pour émettre une lumière ayant une intensité comprise dans une plage de 50 mW/cm² à 150 mW/cm².

6. Dispositif (100) selon la revendication 1,
**caractérisé en ce que**
le dispositif de support (150) est conçu comme une pièce à main à tenir d'une main par un praticien pour le traitement de la région de tissu (W).

7. Dispositif (100) selon la revendication 1,
**caractérisé en ce que**
le dispositif de support (150) est conçu sous forme de tente pour recevoir l'être humain ou l'animal.

8. Dispositif (100) selon la revendication 1,
**caractérisé en ce que**
le dispositif de support (150) est conçu comme un masque respiratoire pour la respiration de l'être humain ou de l'animal.

9. Dispositif (100) selon la revendication 1, la cartouche (200) comprenant
a. un premier réservoir (210) contenant la première composition,
b. un deuxième réservoir (220) séparé de manière étanche aux liquides du premier réservoir (210) et contenant la deuxième composition, et
c. un troisième réservoir (230) séparé de manière étanche aux liquides du premier réservoir (210) et du deuxième réservoir (220) et contenant la troisième composition.

10. Dispositif (100) selon la revendication 9
**caractérisé en ce que**
la cartouche (200) comporte un premier orifice de prélèvement (211) pour prélever la première composition du premier réservoir (210), un deuxième orifice de prélèvement (221) pour prélever la deuxième composition du deuxième réservoir (220) et un troisième orifice de prélèvement (231) pour prélever la troisième composition du troisième réservoir (230), au moins l'un des orifices de prélèvement (211, 221, 231) étant fermé par une valve.

11. Dispositif (100) selon la revendication 1 comprenant la première composition, la première composition étant sous forme de gel et comprenant les composants suivants :
a. de la curcumine dans une proportion en masse de 0,1 % à 10 % de la première composition,
b. Rose Bengale dans une proportion en masse de 0,01 % à 1 % de la première composition,
c. une composition gélifiante et
d. de l'eau.

12. Dispositif (100) selon la revendication 11,
**caractérisé en ce que**
la composition gélifiante contient
a. un poloxamère dans une proportion en masse de 0,2 % à 20 % de la première composition,
b. du 1,2-propylèneglycol dans une proportion en masse de 0,13 % à 13 % de la première composition,
c. 2-propanol dans une proportion en masse de 0,13 % à 13 % de la première composition, et/ou
d. des triglycérides à longueur de chaîne moyenne dans une proportion en masse de 0,05 % à 5 % de la première composition.
